(19) Europäisches Patentamt
European Patent Office
Office européen des brevets

(11) EP 1 978 087 A1

(12) EUROPEAN PATENT APPLICATION

(43) Date of publication:
**08.10.2008 Bulletin 2008/41**

(51) Int Cl.:
***C12M 1/34*** *(2006.01)*

(21) Application number: **07105509.9**

(22) Date of filing: **02.04.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Consultatie Implementatie Technisch Beheer B.V.**
**7201 JB Zutphen (NL)**

(72) Inventor: **The designation of the inventor has not yet been filed**

(74) Representative: **van Loon, C.J.J. et al**
**Vereenigde**
**Johan de Wittlaan 7**
**2517 JR Den Haag (NL)**

(54) **System and method for detecting micro-organisms**

(57) The invention relates to a system for detecting a micro-organism. The system comprises a cap for placement on a vial; the vial to be used for holding a broth for culturing a micro-organism; and a detector operatively associated with said cap. The detector is arranged for detecting, over time, a concentration of predetermined substance. A processing unit is coupled to said detector; for associating said detected substance concentration to a predetermined micro-organism.

EP 1 978 087 A1

## Description

The invention relates to a system for detecting micro-organisms. In addition, the invention relates to a method of testing a substance associated with a predetermined micro-organism.

In the art, in order to detect a micro-organism such as a (pathogenic) bacteria or fungus, first a culture is provided, for example by fermentation. This can be done directly from an inoculation, which is delivering a sample through a septum in a vial by means of a syringe. It is also common practice to first derive pure strains by growing on an agar plate and using a pure colony as the inocculum for the fermentation.

Analysis can be performed visually when pure colonies are used as derived from an agar plate, or using some kind of measurement method at the end of a fermentation process. In the latter case, mostly culture broths that benefit specific bacteria strains are used in combination with a growing-mass method such as evolution of CO2 or assimilation of 02. The CO2 concentration can be measured optically or via pressure. In addition, it is common practice to first obtain pure colonies on an agar plate and then use the pure colonies to inoculate a set of fermentation broths in order to further determine the precise strain of bacteria.

However, this process takes a long time, which is often not available. Even then, a risk of multiple infections is present of bacteria and fungi that are hard to discriminate. It would be desirable to have a rapid test available that would deliver initial results very soon.

According to an aspect of the invention; there is provided a system for detecting a micro-organism, said system comprising: a cap for placement on a vial; the vial to be used for holding a broth for culturing a micro-organism; a detector operatively associated with said cap; wherein the detector is arranged for detecting, over time, a concentration of a predetermined substance and a processing unit coupled to said detector; for associating said detected substance concentration to a predetermined micro-organism or class of micro-organisms.

Throughout the application, it is meant to encompass with "predetermined class of micro-organisms" a class of organisms that may be relevant in a clinical sense for discriminatory purposes.

Preferably, the detector comprises a semiconductor sensor on a micro-hotplate where chemical reactions occur of the traces to be detected. Such detector exploits the variation of electrical resistance of the sensor while, at a certain heating temperature, redox reaction take place on the surface of the sensor. Such hotplate technology is very to variations of the temperature and it is therefore preferably, detection is provided of the traces at a prefixed temperature. In particular, the heater resistance is temperature dependent, which implies that current adjustments may be provided to provide a stable temperature. This can be done by a balancing circuit which balances the heat resistor to a predefined resistor value.

US patent No 4,847,783 discloses a balancing circuit comprising an adjustable resistor for tuning the heater element to a predefined resistor value.

The invention will now be clarified on the basis of non-limiting exemplary embodiments represented in the appended drawing. In the drawing:

Figure 1 shows two exemplary arrangements embodying an aspect of the invention;
Figure 2 shows an illustrative graph of a growth test of a pseudomonas bacterial culture on a broth;
Figure 3 shows another illustrative graph of a growth test of an E.Coli bacterial culture on a broth;
Figure 4 shows a typical layout of the gas sensor incorporated in the embodiments of Figure 1;
Figure 5 shows a response characteristic of a heatable metal oxide sensor that is exposed to a variety of compositions or chemical substances in varying concentrations;
Figure 6 shows measured resistance - temperature diagrams of three hotplate sensors;
Figure 7 shows a preferred embodiment of the inventive concept;
Figure 8 shows power temperature relationships for the same heater elements as in Figure 5; and
Figure 9 shows a characteristic temperature response for a variety of sensed substances during a thermal detection cycle.

Figure 1 shows two exemplary embodiments of the present invention. Although an alternative embodiment is feasible (not shown) wherein, in a closed system, a pump draws the headspace gas via a filter over sensors and returns the headspace gas back to the headspace, according to an aspect of the invention a special cap 1 is provided that can be placed on the fermentation bottle or vial 2. The cap 1 comprises a detector 3 operatively associated with said cap 1. Typically, the detector 3 may contain the sensors, electronics, communication (RF) and microprocessor to provide a stand-alone application.

In this embodiment, the sensors will measure the headspace 6 over the broth 7 provided in the vial 2 via simple diffusion without any mechanical pump.

The detector 3 in Figure 1A comprises a puncturing device 4 for puncturing a septum 5 provided on said vial 2. This detector 3 can be recycled by sterilizing and/or replacement of the puncturing device 4.

In Figure 1B, the cap 1 and the detector 3 are provided integral. In this embodiment, the cap 1 comprises a septum 5 to be punctured while inserting a micro-organism test substance through inoculation.

A detector unit shown in Figure 1 has distinct advantages over other detection systems such as pressure/optical CO2 measurement and colorizing reactions. The detection level of the e-nose is in the order of magnitude of several ppm, and the detector also makes distinction of the gas composition possible at a very early stage. In addition, in contrast to the present invention, colorizing reactions and monitoring of $CO_2/O_2$ production/consumption require large amounts of reactants or fermentation times.

In Figure 2 an illustrative graph of a growth test of a pseudomonas bacterial culture on a broth.

In Figure 3 another illustrative graph of a growth test of an E.Coli bacterial culture on a broth is shown.

The tests of Figure 2 and Figure 3 were performed at relatively low ambient temperature of 18 degrees. At optimal temperatures the growth times can be expected to shorten considerably, for example, so that a test can be completed within 3-4 hours.

Turning to Figure 3 conductivity outputs of a palladium sensor (upper two lines 30 and 31) and a platinum sensor (lower two lines 32 and 33) were measured. The upper two lines 30, 31 represent measured maxima; minima respectively, measured in time during a temperature cycle of the palladium detector. The "snap-shots" 35 and 36 are showing normalized graphs of temperature cycles on a specific moment in time for platinum and palladium sensors respectively; that is three hours after start. Likewise snap-shots 37 and 38; resp 39 and 40 are measured temperature cycles after 10 hours and after 20 hours respectively; for platinum and palladium sensors respectively.

Accordingly, over time a substance concentration in said vial indicative of the presence or absence of a predetermined micro-organism is detected through the conductivity measurement.

The tests demonstrate the distinct differences between pseudomonas and E. Coli both in quantitative signal over time (notice the temporary 'dip' of E.Coli), and the shape of the patterns of the e-nose at specific times (the snap-shot inserts). The tests were performed with the same broth. It can thus be seen that a detected substance concentration can be associated to a predetermined micro-organism or class of micro-organisms.

Accordingly, the method shows that a substance associated with a predetermined micro-organism or class of micro-organism can be tested that may be relevant to discriminate for clinical purposes. The substance can be a nutrient and/ or metabolite of said micro-organism. Typically the micro-organism is a bacterial or fungal pathogen, to be tested for in clinical purposes. For instance, as a nutrient, nitrogen, sulfur or carbon containing substances may be added. Depending on a measured (metabolic) response, a classification can be made of the micro-organisms to be tested.

The continuous measurements of the detector enable continuous tracking of the headspace and thus enable following the metabolizing reactions over time. By careful selection of the composition of the broth, the monitoring over time allows following the sequential changes in metabolizing reactions/pathways as nutrients are depleted in preferred order. For example, a broth containing nutrients A, B and C will exhibit different changes in headspace over time for bacteria that have different preferences (or capabilities) for those nutrients.

By taking the combination of all patterns measured during the growth over time a distinct 'fingerprint' is obtained that is far more detailed and specific than a single snap-shot measurement at the end of the fermentation process, as is current practice as published in literature.

Turning to Figure 4 a typical layout is shown for a detection unit, in particular detector 101 implementing a heatable conducting plate 102, also known as hotplate sensor 102. The hotplate sensor 102 is typically provided by a metal oxide sensor element 103 which is sensitive to chemical reactions taking place near the sensor surface area, that is in close spatial relationship with a heater element 104. This sensor element 103 shows in particular a variation in conductance depending on chemical traces reacting near the exposed surface area 105 thereof. Various metal oxide sensor elements 103 are known, including but not limited to tin oxide, zinc oxide, iron oxide and tungsten oxide sensors with or without added catalyst, including but not limited to platinum and palladium.

The hotplate 102 is heated by a heater element 104 which is preferably attached in close vicinity of the sensor element 103 produced by MEMS (micro electrical mechanical systems) technology thus ensuring an identical temperature of the conducting sensor element 103 and the heater element 104. The heater element 4 has a low thermal mass and is controlled by a processor 6 for to provide a stabilized temperature in said sensor element 103. Typically this is provided by a balancing circuit implementing a Wheatstone bridge as will be further elucidated in Figure 6.

Furthermore, the sensor element 103 is connected to a detection circuit 107 for detecting a change of resistance in the sensor element 103 in accordance with the presence of a chemical trace reacting in the presence of the conducting plate. The output of the detection circuit 107 in connection with a preset temperature provided by the processor 106 are stored in an internal memory element 108 of the detector, which can be any type of memory , typically a flash memory.

In the memory element 108, among others, a plurality of detected resistance values in the detection circuit relative to a plurality of preset temperatures can be stored to form a footprint of a number of chemical substances 109 which are sensed by the hotplate 102 by exposing the hotplate to a flow of gas 110. Alternatively, the hotplate can be subjected to stagnant air.

In the embodiment shown, the results are stored in the memory element 108 to be transmitted via a communication terminal 111 to a base station 112 comprising a database for storing footprints of predetermined chemical substances. Thus the stored footprints can be communicated to the base station 112 comprising a database 113, for providing a best match 114 of any of said stored footprints in the memory element 108 to any of footprints stored in the database 113 of known chemical substances. In this way a particular detected composition of chemical substances can be identified in the database 113 via per se known pattern recognition and identification software techniques.

Although in this embodiment, the identification of a sensed chemical composition can be done online or offline in an external base station 112, the detector 101 may also be equipped with specific matching routines which can match the detected footprint with one or more predefined chemical substances on board of the detector 101. In this way, the detector 101 can be easily modified to provide a detector for detecting specific predetermined chemical substances. In this (not shown) embodiment, the detector 101 hence comprises in addition a comparison circuit for comparing a stored footprint with a predermined set of prestored footprints of predetermined chemical substances, so as to determine a particular detected chemical substance.

Figure 4 shows different conductivity responses of the hotplate 102, in particular, for a concentration of 20 and 80 ppm (line 115 and 116 respectively) of toluene and for a concentration of 50 and 100 ppm (line 117 and 118 respectively) of butyl acetate. Also a blank response 119 is shown, illustrating a detected conductance for varying temperatures. The typical detection temperatures vary between 200 and 600 °C. It can be shown generally that the metal oxide sensor produces peak conductance values for different chemical substances on different temperature values and for different peak values. For example, the conductance for toluene is generally higher than for butyl acetate. However, it is clear that when a precise temperature setting is unknown, the discriminatory power between 20 ppm toluene and 100 ppm butyl acetate is poor, even when a test is conducted at various temperatures. Therefore, an accurate setting of the temperature is important for obtaining reliable test results.

Typically, the metal oxide sensor 102 is sensitive for oxygen reducible substances. Typically, components show maximum conductance according to particular temperatures settings. By obtaining the detection results at various temperature, a footprint can be obtained of the variety of chemical substances. This footprint can be compared to a number of footprints of known pure substances or mixtures that are stored in a database 113 as referred to in Figure 4.

Figure 5 shows a measured resistance - temperature diagram of the heater element 104. As will be further elucidated with reference to Figure 7 the heater element 104 can be integrated in a balancing circuit to preset the resistor value thereof to a predetermined value. Thus, a balancing circuit can provide a preset resistor value of the heater element 104, giving rise to a predetermined temperature according to the resistance - temperature diagram shown in Figure 6.

However, the diagram in Figure 6 clearly shows that the temperatures of the hotplate 102 are varying substantially for a preset resistor value. For three hotplates W1, W2, W3 shown, the hotplates W1 and W2 are of a same type. This means that the macroscopic dimensions of the heater elements 104 are almost the same. Nonetheless, where the resistance varies only 1.5 Ohm at room temperature, at a preset resistance of 160 Ohm a difference of 25 ° C is provided by the heater element. It shows that without individual calibration of the heater element 104, presetting the heater element 104 to a fixed resistance can give an unacceptable spread in temperatures, which affects the reliability of the detector 101.

Figure 7 shows a preferred embodiment of the inventive concept. In particular, Figure 7 shows a processor 106 and a balancing circuit 120 having an adjustable resistor 121 for tuning the heater element 104 to a predefined resistor value.

The balancing circuit 120 comprises essentially a Wheatstone bridge arrangement of fixed resistors R5, R6, R7, R8, in combination with a heatable resistor 4(also indicated in the drawing as RH) and a tunable digital potentiometer which functions as the adjustable resistor 121 (also indicated in the drawing as U10). The digital potentiometer 121 has a very good linearity. The resistance in the bridge circuit 120 is determined by the resistor R8 circuited parallel to the digital potentiometer 121. This resistor R8 (as well as the other fixed resistors R5, R6 and R7) has a very precise resistive value, typically with a margin of error of less than 0.1 %. The circuit is balanced by the operational amplifier 122 (U11) which controls the voltage across the heater element 104. In particular, the amplifier U11 will control the Voltage between the + and - terminals of the amplifier so that there is no voltage difference, i.e. so that the bridge is balanced. When the Voltage difference is higher, the current through the heater element 104 (RH) will increase. The heater element 104, conducting an increased current, will heat up and the resistance will rise accordingly. Accordingly a preset resistive value of the heater element 104 can be controlled, wherein the resistive value of the heater element 104 is known expressed as a ratio of resistive values of the R5, R6, R8, and a fraction of R7 determined by tunable digital potentiometer 21 (also indicated in the drawing as U10)

In addition, Figure 7 shows a test circuit 123 for the balancing circuit 120 for measuring a dissipated power in the heater element 104 and for calculating a real temperature from the dissipated power in the heater element 104 based on the predetermined power - temperature characteristic which will be further elucidated with reference to Figure 8.

In this embodiment the test circuit 123 comprises a pair of test terminals 124 (one being grounded) that directly connect to the terminals of the heater element 104. This arrangement provides a conventiently implementable circuit 121 for calculating the power dissipation in the resistor using the familiar formula $V_H^2/R_H$ with $V_H$ being a detected voltage difference across the heater element 4. In addition, $R_H$ indicates a true resistive value of the heater element 104 derived from the balancing circuit 120.

In one embodiment, the test circuit 123 comprises a calculating circuit 125 to calculate an offset value for the digital potentiometer 121. In particular, the test circuit 123 comprises a switch 126 to activate the calculating circuit 125. In this embodiment, the test circuit 123 measures a dissipated power in predefined neutral conditions.

Upon activation, a method of calibrating the hotplate chemical trace detector 101 is carried out. In particular, using the test circuit 123 there is provided a predetermined power level to the hotplate 102 by adjusting the adjustable resistor 121. When placing the sensor is placed in a neutral ambiance the predetermined power level can be related to a set temperature using a known power - temperature characteristic of the heater plate. Thus, a precise set-point for a predetermined number of temperatures can be provided to the processor 106 for the heater element 104, thus zeroing the adjustable resistor 121 to a preset value relating to the set temperature.

In another embodiment, the test circuit is connectable to a calibration circuit for providing a lookup table to the processor 106 for calculating preset resistor values so as to provide predetermined real temperatures to said heater element. In this embodiment, the detector 101, in particular, the processor 106, may be attached to a separate a test circuit 123, for instance, in a factory setting, indicated by the dotted lines 127. In predefined neutral conditions, a series of predetermined power settings to the heater element 104 is provided by adjusting the adjustable resistor 121. Accordingly a series of predetermined temperatures to these power settings is provided using the power - temperature characteristic of the heater plate. In this way a series of setpoints for setting a temperature can be provided to form a lookup table to the adjustable resistor 121 for providing preset resistor values so as to provide predetermined real temperatures to said heater element 104. The lookup table is then integrated in the processor 106, in particular, is provided in a local memory to be accessed when setting the adjustable resistor to a predetermined temperature setting.

With the hotplate chemical trace detector as hereabove described, a precise temperature of the heater element 104 can be measured by the test circuit 123, without having to rely on the resistance - temperature characteristic of the heater element that may vary from sample to sample. In particular, a precise set point for the heater element can be provided.

Thus, when using this setpoint, a temperature can be set by adjusting the resistor in the balancing circuit to a real known temperature. The amount of power to achieve this temperature can be related to a dissipated reaction energy of the chemical trace. Indeed, the calculating circuit 125 can be arranged to calculate a difference of a measured input power from the test terminals 124 and a calculated input power. This calculated input power can for instance be provided using the known real temperature derived from the preset resistor value 121 after calibration and relating it to a calculated power in the heater element 104 using the power - temperature characteristic of the heater element 104.

In this way, a new way of characterizing chemical substances can be provided, whereas, in addition to a measured conductance of the sensing element 103.

In another embodiment, a dynamic temperature modulation is used of the hotplate 102. In this embodiment, the processor 106 is arranged to provide a sliding temperature to the heater element 104. Thus, by providing a predefined dynamic temperature profile to the heater element 104 and deriving a sensed conductance of the sensor element 103, more information can be collected from the sensor to provide it to pattern recognition software implemented in the database 113, which for this purpose stores conductance diagrams of predefined chemical substances measured in standard conditions as a function of known real temperature and temperature dynamics.

Figure 8 shows a power - temperature characteristic for two macroscopically identically hotplate sensors 102. The term macroscopically identical indicates a generally identical geometric structure for the hotplate 102, that is, a generally identical conducting structure for conducting heat from the heater element 104 and the sensor element 103. The power - temperature characteristics for the two heater elements W1 and W2 appear to be substantially identical although heater element W1 shows a resistance of 88.1 Ohm at 22.3 °C and heater element W3 shows a resistance of 97.4 Ohms at 22.1 °C, a difference of more than 10%. The power - temperature characteristic is valid in standard conditions, at room temperature in clean air. In non-standard conditions the actual temperature can be measured and used for recalculating the power-temperature characteristic. In this way, the temperature of the heater element 104 $T_{sensor}$ can be derived for a predetermined number of settings of the digital potentiometer 121 $R_{pot}$. This provides a gauge line which can be converted to a function using a linear regression.

$$\mathrm{Rpot} = \mathrm{F(Tsensor)} \qquad [1]$$

This equation can be implemented in software operating the processor 106 so that a temperature can be preset with a deviation which may be less than 3 - 5 °C.

Figure 9 shows a detector response for a thermal cycle, that is, for a detector that is arranged to increase a sensing temperature of the detector, in particular, of the hotplate 102 from substantially 100 °C to substantially 600 °C while measuring a sensor response. To this end, a normalized amplitude is shown on the Y-axis of the graph; wherein a thermal cycle is passed by a temperature that varies substantially sinusoidal in time. This response is preferably fixed within a predetermined time frame, of substantially a few seconds, typically from about 5 to 15 seconds.

A normalized amplitude can be a good measure for a typical detector response to various gaseous substances; responses are shown for various substances, in particular, Xylene, Methylamine, Formic Acid, NH3 and H2S. For reference, a blanc response is also shown.

Due to dimensional differences, and also due to varying gas concentrations, a typical response will vary from detector to detector. By normalizing the amplitude the amplitude can be made substantially invariant, so that a temperature cycle form is indicative of a typical substance, irrespective of dimensioning and concentration variations.

In another embodiment, a non-normalized amplitude can be made indicative of a gas concentration, provided it is calibrated.

By providing a predetermined temperature, the curve as depicted in Figure 9 will not vary due to temperature differences, so that good comparison can be made with prestored temperature responses.

Thus, by providing temperature calibrated detectors, a detected temperature response can be normalized and then analyzed. The presence (typically, irrespective of concentration) of a particular gaseous substance can then be indicative for a filter wear out.

Although the thermal cycle response of Figure 9 is a result of a sinusoidal temperature variation, other types of variations can be applied as well, such as linear variation or block variations etc. The loop hystereses of the loop, which is related to a loop form, will be typically time dependent, so that the temperature variation is preferably kept fixed for ease of comparison. A typical repetitive temperature variation will result in a generally repetitive detector response, so that, in particular, for continuous sinusoidal temperatuurcycles subsequent measurement cycles will align.

In conclusion, the loop hysteresis, and thus a temperature cycle response will be a function of gaseous substance and the time cycle (limit value and change velocity) of temperature variation.

Although the invention has been set forth using a limited number of embodiments the skilled person will appreciate that various modifications and adaptations thereto are possible without departing from the scope of the invention. For instance, it is possible to derive the power dissipated in the heater element by a test circuit 123 coupled more indirectly to the heater element, for instance a terminal that measures the output voltage of the amplifier U11 of Figure 6. Alternatively, or in addition the test circuit 123 that does not need to use the balancing circuit 120 but could measure the resistance of the heater directly using a preset value of the digital potentiometer 121.

The test method can be performed in-field, such as field labs (development countries, crises areas), at general practitioners (GP's, huisarts), in industry (salmonella, shigalla, legionella), dairy industry etc. Fast and cheap tests aimed at rapid screening of pathological bacteria for hospitals is also possible.

The invention is not limited to the disclosure of the embodiments shown in the description but encompasses variations and modifications thereto and is determined by the scope of the annexed claims and their equivalents.

## Claims

1. A system for detecting a micro-organism, said system comprising:

   - a cap for placement on a vial; the vial to be used for holding a broth for culturing a micro-organism;
   - a detector operatively associated with said cap; wherein the detector is arranged for detecting, over time, a concentration of a predetermined substance and
   - a processing unit coupled to said detector; for associating said detected substance concentration to a predetermined micro-organism or class of micro-organisms.

2. A system according to claim 1, said processing unit further comprising a memory for storing a plurality of detection values over time to form a footprint indicative of the presence or absence of a predetermined micro-organism associated with said footprint.

3. A system according to claim 2, said processing unit further comprising a database storing footprints of predetermined micro-organisms and their associated substances; and a comparison circuit for comparing a best match of said footprint to any of said stored footprints so as to determine a particular detected micro-organism.

4. A system according to claim 1, wherein the detector is provided with a puncturing device for puncturing a septum provided on said vial.

5. A system according to claim 1, wherein the cap and the detector are integral; the cap further comprising a septum to be punctured while inserting a micro-organism test substance.

6. A system according to claim 1, wherein said detector functions at a detection temperature higher than 100 °C.

7. System according to claim 6, wherein said detector is arranged to vary, within a predetermined time frame, said detection temperature in a range between 100 °C and 600 °C while measuring a response.

**8.** A system according to claim 1, **characterized in that** the detector comprise multiple sensors.

**9.** A system according to any one of the preceding claims, **characterized in** the detector comprises a metal oxide semiconductor (MOS) sensor.

**10.** A system according to any one of the preceding claims, **characterized in that** the system comprises a processing unit for comparing the detected concentrations of the component in the various flows.

**11.** A system according to any one of the preceding claims, **characterized in that** the system comprises a transmitter comprised in the detector, for communicating collected data to a remotely positioned receiver.

**12.** A system according to claim 1, wherein said detector comprises:

- a heatable conducting plate comprising a heater element having a predetermined power - temperature characteristic;
- a balancing circuit comprising an adjustable resistor for tuning the heater element to a predefined resistor value;
- a processor for adjusting the adjustable resistor so as to provide a stabilized temperature in said heatable conducting plate; and
- a detection circuit for detecting a change of resistance in the heatable conducting plate in accordance with the presence of a chemical trace reacting in the presence of the conducting plate.

**13.** A system according to claim 12, wherein said heatable conducting plate comprises palladium or platinum.

**14.** A system according to claim 12; further comprising

- a test circuit for measuring a dissipated power in the heater element and for calculating a real temperature from the dissipated power in the heater element based on the predetermined power - temperature characteristic.

**15.** A system according to claim 14, wherein the test circuit is connectable to a calibration circuit for providing a lookup table to the processor for providing preset resistor values so as to provide predetermined real temperatures to said heater element.

**16.** A system according to claim 14 or 15, wherein the test circuit is coupled to a processor to calculate a dissipated reaction energy of the chemical trace as a difference in a measured input power and a calculated input power from the preset resistor value after calibration.

**17.** A system according to any of the claims 14, wherein the test circuit comprises a pair of test terminals that directly connect to the terminals of the heater element.

**18.** A method of testing a substance associated with a predetermined micro-organism,wherein a detector is operatively associated with a cap for placement on a vial; the vial to be used for holding a broth for culturing a micro-organism; comprising:

- detecting, over time, by the detector, a substance concentration in said vial indicative of the presence or absence of a predetermined micro-organism; and
- associating said detected substance concentration to a predetermined micro-organism.

**19.** A method according to claim 18 further comprising

- detecting a substance concentration at a detection temperature higher than 100 °C over time to form a footprint indicative of the presence or absence of a predetermined micro-organism associated with said footprint; and;
- comparing a best match of said footprint to any of a number of stored footprints of predetermined micro-organisms and their associated substances.

**20.** A method according to claim 18, further comprising: providing, over time, a plurality of predetermined nutrients; the decrease thereof and/or the increase of an associated metabolite being indicative of a predetermined micro-organism.

**21.** A method according to claim 20, wherein the substance is a nutrient and/ or metabolite of said micro-organism, said micro-organism being a bacterial or fungal pathogen.

**22.** A method according to claim 18, wherein said detection temperature is varied in a range of 100 °C to 600 °C within a predetermined time frame.

Figure 1

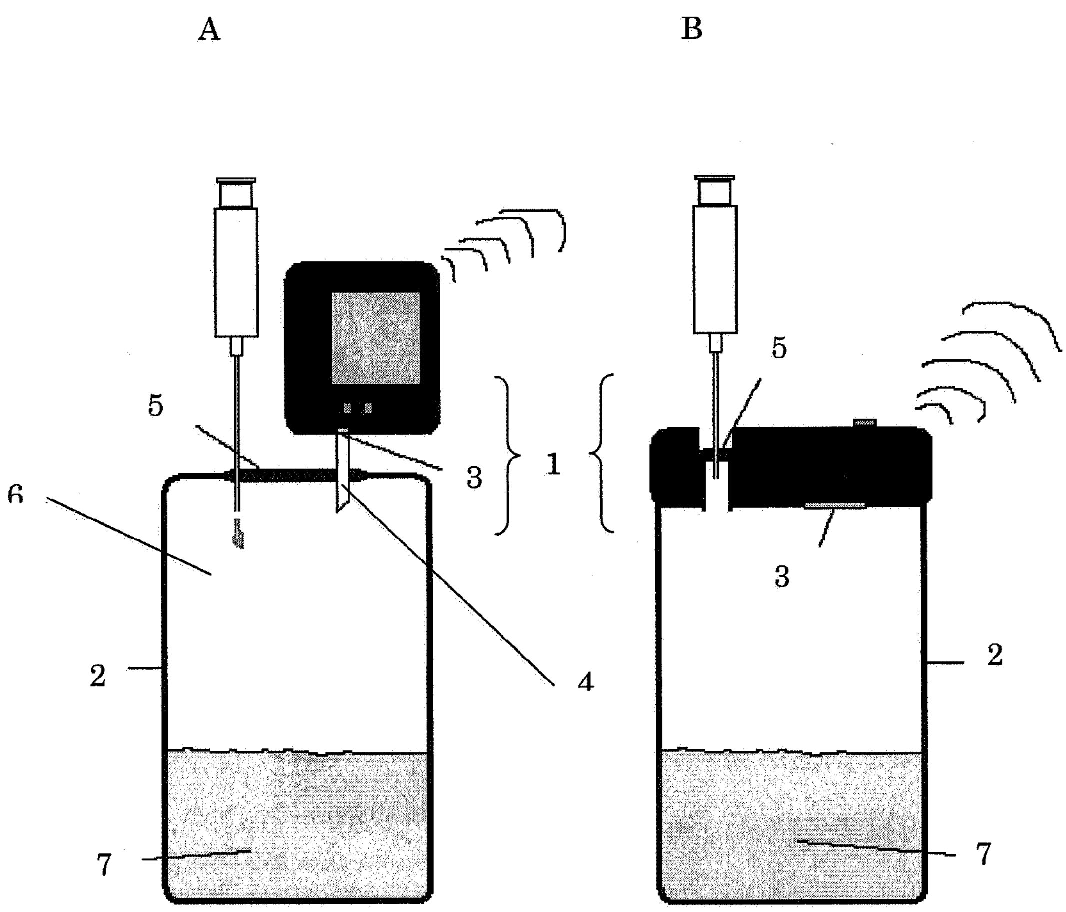

Figure 2

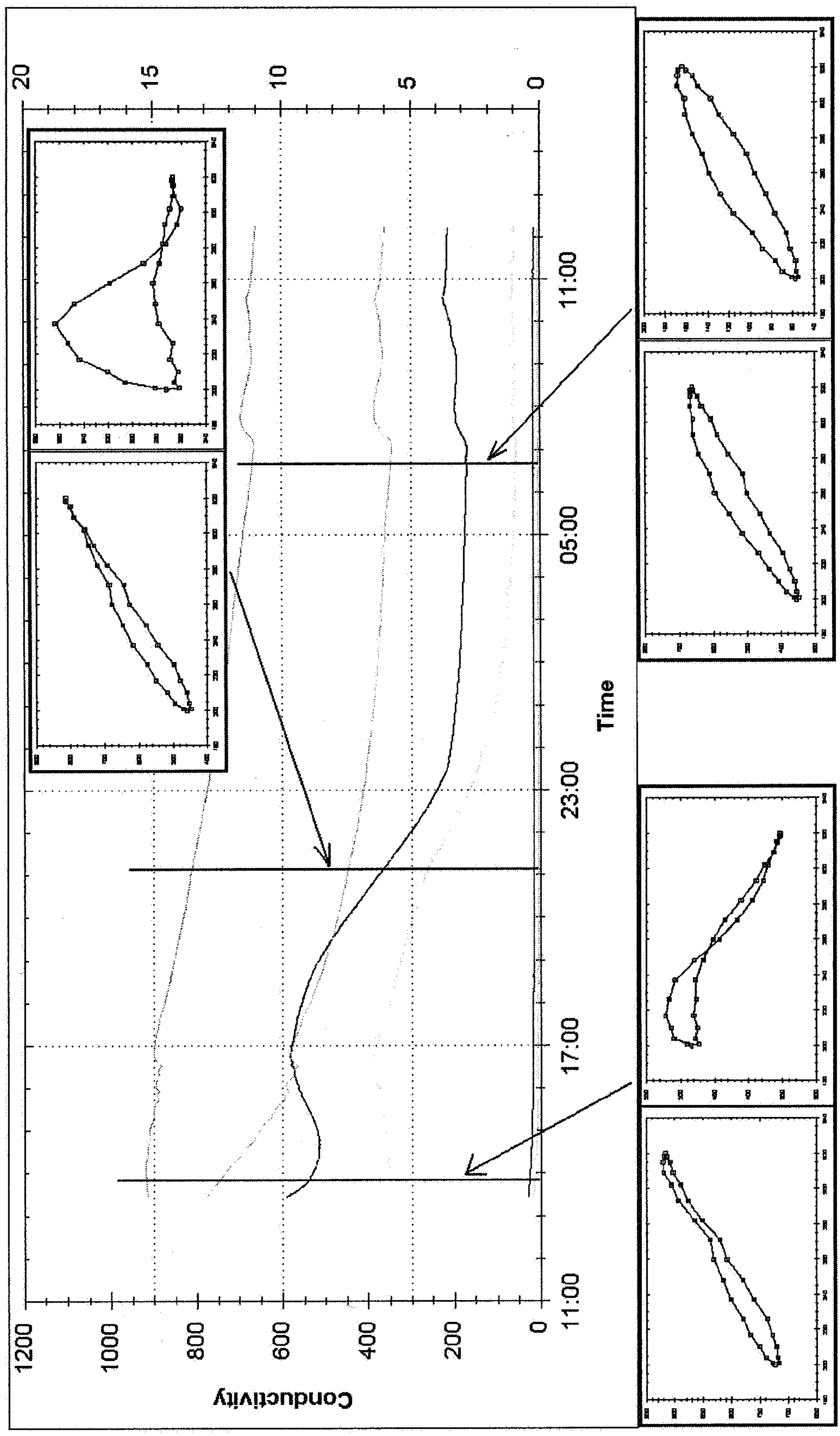

Conductivity
1200
1000
800
600
400
200
0
20
15
10
5
0
11:00
17:00
23:00
05:00
11:00
Time

Figure 3

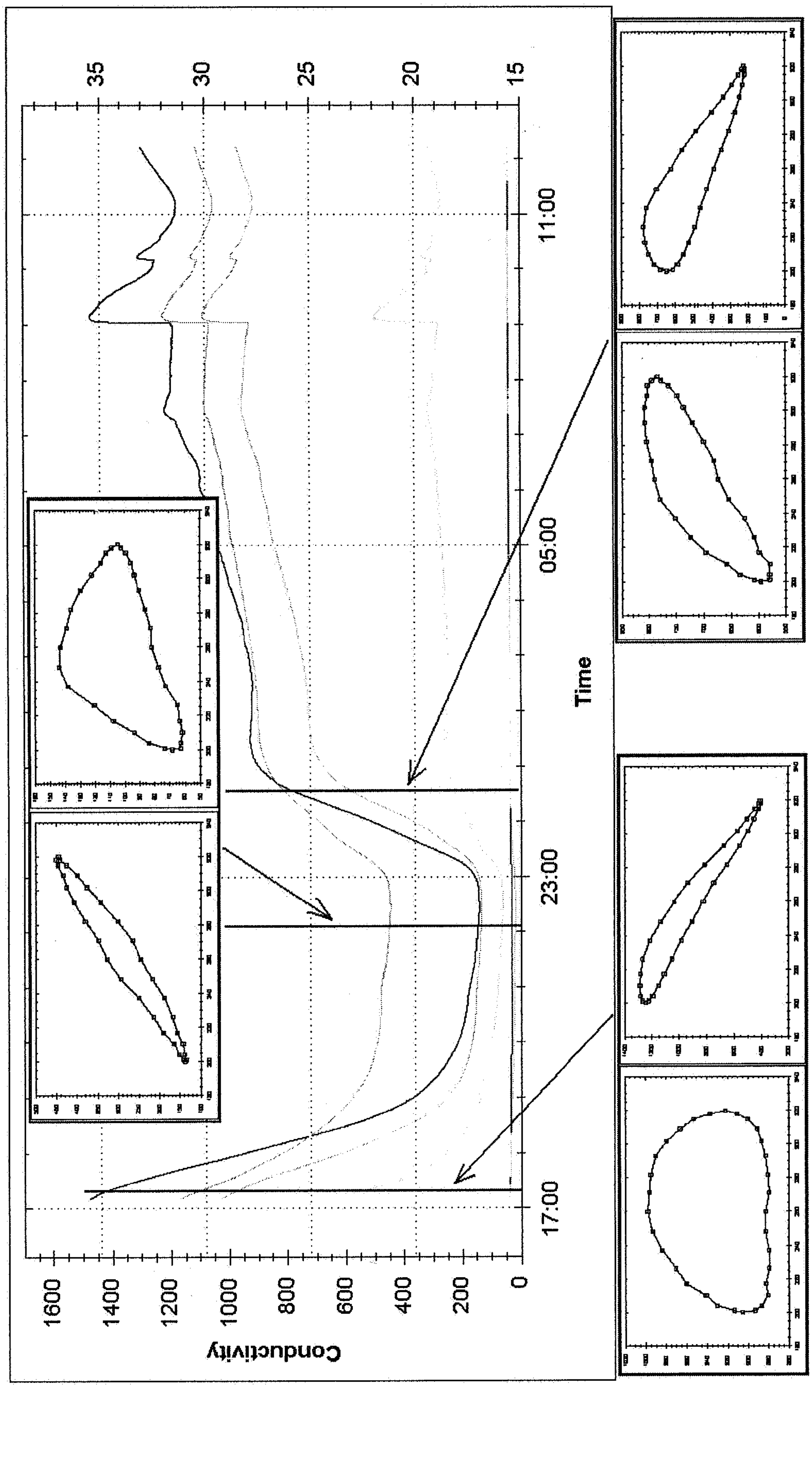
Conductivity
0
200
400
600
800
1000
1200
1400
1600
Time
17:00
23:00
05:00
11:00
15
20
25
30
35

Figure 4

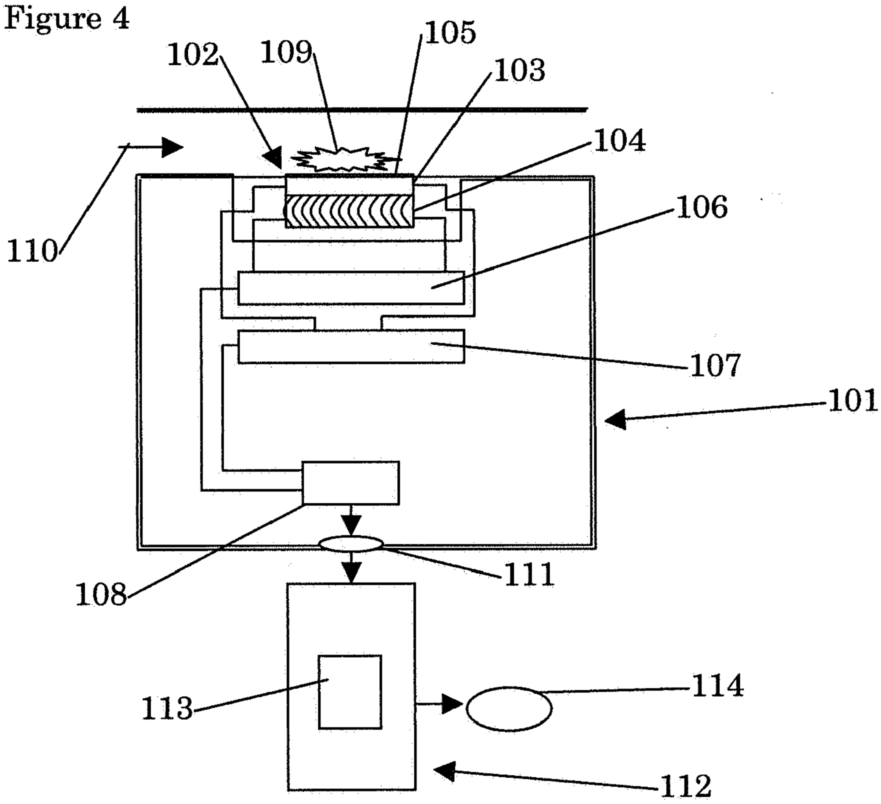


Figure 5

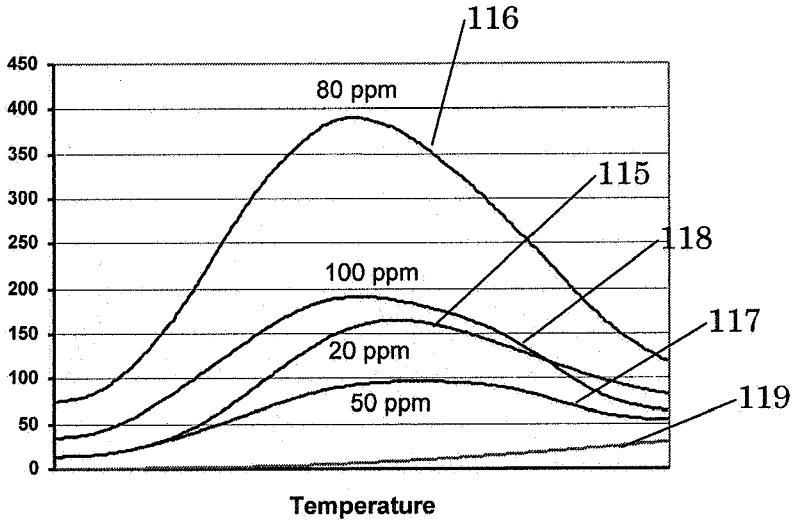

Figure 6

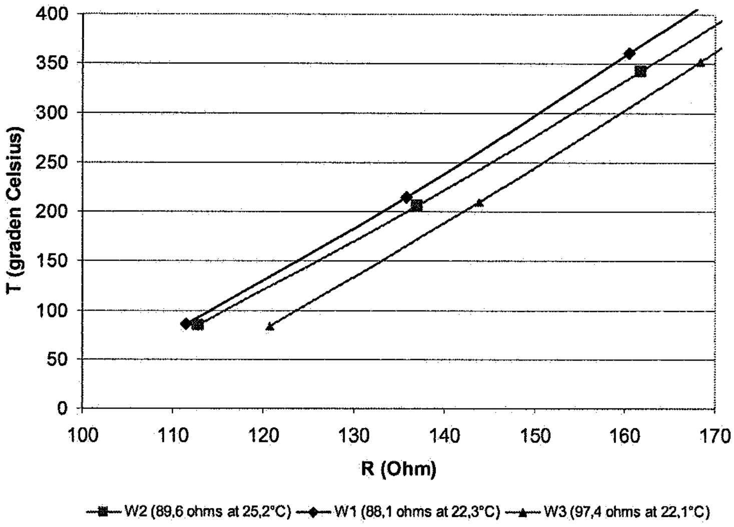


Figure 7

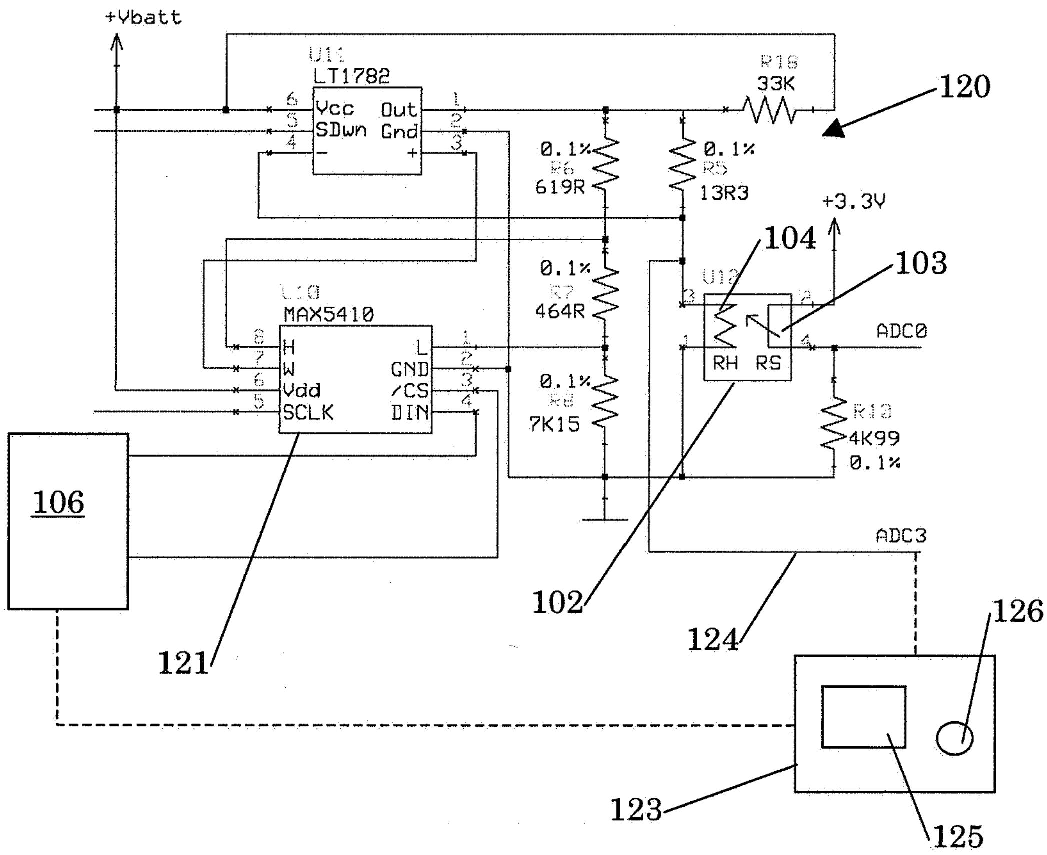

Figure 8

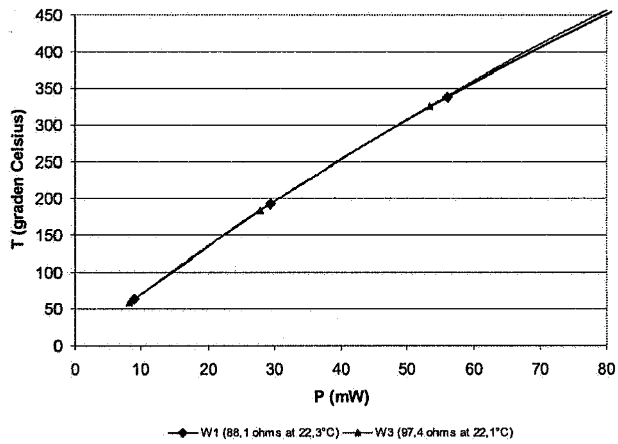
450
400
350
300
250
200
150
100
50
0
T (graden Celsius)
0
10
20
30
40
50
60
70
80
P (mW)
W1 (88,1 ohms at 22,3°C)
W3 (97,4 ohms at 22,1°C)

Figure 9

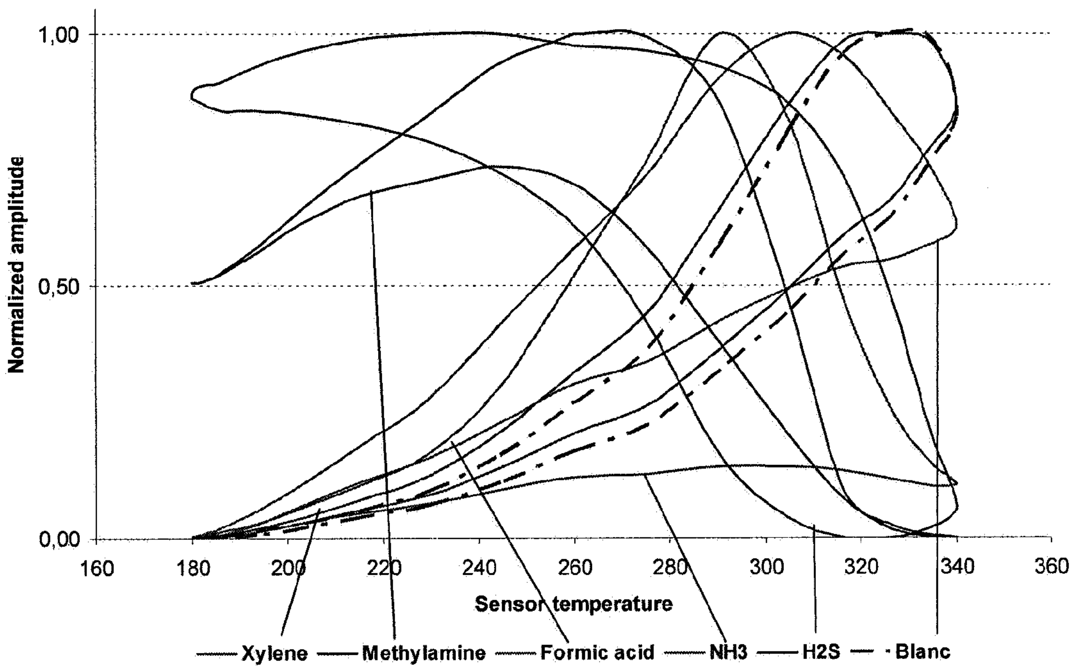
1,00
0,50
0,00
Normalized amplitude
160
180
200
220
240
260
280
300
320
340
360
Sensor temperature
Xylene
Methylamine
Formic acid
NH3
H2S
Blanc

European Patent Office

# EUROPEAN SEARCH REPORT

Application Number
EP 07 10 5509

| Category | Citation of document with indication, where appropriate, of relevant passages | Relevant to claim | CLASSIFICATION OF THE APPLICATION (IPC) |
|---|---|---|---|
| A | WO 2006/079846 A (GRAF INTERNAT LTD [GB]; FRANCESCO GINO [GB]) 3 August 2006 (2006-08-03)<br>* page 1, lines 4-16 *<br>* page 2, lines 3-5 *<br>* page 4, lines 18-21 *<br>* page 5, lines 1-7,18-27 *<br>* claims; figures * | 1-22 | INV. C12M1/34 |
| A | WO 03/091718 A (SIEMENS AG [DE]; BURGHARDT THOMAS [US]) 6 November 2003 (2003-11-06)<br>* page 1 - page 3 *<br>* claims; figures * | 1,18 | |
| A,D | US 4 847 783 A (GRACE RICHARD [US] ET AL) 11 July 1989 (1989-07-11)<br>* column 1, lines 6-12 *<br>* column 2, lines 40-68 * | 1,18 | |
| A | WO 02/50528 A (EIDGENOESS TECH HOCHSCHULE [CH]; BALTES HENRY [CH]; BARRETTINO DIEGO [) 27 June 2002 (2002-06-27)<br>* page 1, lines 7-16 *<br>* page 3, line 31 - page 4, line 7 *<br>* page 5, line 27 - page 6, line 2 *<br>* claims; figures * | 1,18 | TECHNICAL FIELDS SEARCHED (IPC)<br>C12M<br>C12Q<br>G01N |
| A | BARRETTINO D ET AL: "HOTPLATE-BASED CONDUCTOMETRIC MONOLITHIC CMOS GAS SENSOR SYSTEM"<br>2003 SYMPOSIUM ON VLSI CIRCUITS. DIGEST OF TECHNICAL PAPERS. KYOTO, JAPAN, JUNE 12 - 14, 2003, SYMPOSIUM ON VLSI CIRCUITS, NEW YORK, NY : IEEE, US,<br>12 June 2003 (2003-06-12), pages 157-160, XP002375702<br>ISBN: 4-89114-034-8<br>* the whole document * | 1,18 | |
| | -/-- | | |

The present search report has been drawn up for all claims

| Place of search | Date of completion of the search | Examiner |
|---|---|---|
| Berlin | 27 August 2007 | Böhm, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

European Patent Office

# EUROPEAN SEARCH REPORT

Application Number
EP 07 10 5509

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 4 638 443 A (KANEYASU MASAYOSHI [JP] ET AL) 20 January 1987 (1987-01-20)<br>* column 1, lines 6-12 *<br>* column 3, lines 3-11 *<br>----- | 1,18 | |
| | | | TECHNICAL FIELDS SEARCHED (IPC) |

The present search report has been drawn up for all claims

1

| Place of search | Date of completion of the search | Examiner |
|---|---|---|
| Berlin | 27 August 2007 | Böhm, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 10 5509

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2007

| Patent document cited in search report | Publication date | Patent family member(s) | Publication date |
|---|---|---|---|
| WO 2006079846 A | 03-08-2006 | NONE | |
| WO 03091718 A | 06-11-2003 | DE 10218834 A1 | 04-12-2003 |
| US 4847783 A | 11-07-1989 | EP 0293255 A2 | 30-11-1988 |
| WO 0250528 A | 27-06-2002 | AU 2235702 A | 01-07-2002 |
| | | EP 1360478 A1 | 12-11-2003 |
| | | US 2004075140 A1 | 22-04-2004 |
| US 4638443 A | 20-01-1987 | JP 4039029 B | 26-06-1992 |
| | | JP 59153159 A | 01-09-1984 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4847783 A